# EUROPÄISCHE PATENTSCHRIFT

(11) **EP 1 278 506 B1**
(45) Veröffentlichungstag und Bekanntmachung des Hinweises auf die Patenterteilung: **19.12.2007**
(21) Anmeldenummer: 01923456.6
(22) Anmeldetag: 27.04.2001
(51) Int. Cl.: A61K 8/19

(54) **ZAHNREINIGUNGSMITTEL**
TOOTH CLEANING AGENT
AGENT D'HYGIENE DENTAIRE

(30) Priorität: 03.05.2000 CH 866002000
(43) Veröffentlichungstag der Anmeldung: 29.01.2003
(73) Patentinhaber: WIGET WIGET & PARTNER GMBH, 8006 Zürich (CH)
(72) Erfinder: WIGET, Ernst, A., CH-8173 Riedt-Neerach (CH)
(74) Vertreter: Troesch Scheidegger Werner AG
(86) Internationale Anmeldenummer: PCT/CH2001/000267
(87) Internationale Veröffentlichungsnummer: WO 2001/082883

(56) Entgegenhaltungen:
- US-A- 3 151 027
- US-A- 5 735 942
- PATENT ABSTRACTS OF JAPAN vol. 1998, no. 06, 30. Juni 1998 (1998-06-30) & JP 10 067627 A (SUNSTAR), 10. März 1998 (1998-03-10)
- PATENT ABSTRACTS OF JAPAN vol. 1998, no. 10, 31. August 1998 (1998-08-31) & JP 10 120540 A (SUNSTAR), 12. Mai 1998 (1998-05-12)
- DATABASE WPI Week 199346 Derwent Publications Ltd., London, GB; AN 1993-365103 XP002174854 & JP 05 271029 A (KOA GLASS KK), 19. Oktober 1993 (1993-10-19)

## Beschreibung

Die vorliegende Erfindung betrifft ein Zahnreinigungsmittel, wie insbesondere eine Zahnpasta für den täglichen Gebrauch, eine Zahnpasta für den monatlichen Gebrauch, ein Zahnreinigungsmittel als Prophylaxepaste sowie ein Verfahren zum Reinigen der Zähne.

Zahnreinigungsmittel, wie beispielsweise Zahnpasten, sollen Nahrungsmittelreste, Plaque, unverkalkte Zahnbeläge und Verfärbungen entfernen und gleichzeitig dem Konsumenten ein erfrischtes Mundgefühl geben. Dabei sollen die Zähne nicht nur gereinigt, sondern auch poliert (strahlendes Lachen) werden. Zusätzlich soll eine Zahnpasta Wirkstoffe enthalten, die Karies und Gingivits verhüten. Grundsätzlich können Zahnreinigungsmittel in drei Gruppen unterteilt werden:
1. Zahnpasten für den täglichen Gebrauch
2. Zahnpasten für den wöchentlichen oder monatlichen Gebrauch in Ergänzung zu Zahnpasten unter 1, da stärker wirksam, und
3. Prophylaxepasten für den halbjährlichen Gebrauch.

Zahnpasten gemäss Gruppe 2 stellen eine Ergänzung zu der Verwendung von herkömmlichen Zahnpasten dar und werden insbesondere zur Beseitigung von hartnäckigen Zahnverunreinigungen und von Zahnbelag bzw. von Plaque verwendet. Diese Zahnreinigungsmittel werden in Abständen von ca. einigen Wochen bzw. beispielsweise monatlich verwendet und sind eine wichtige Ergänzung zum täglichen Zähneputzen zur Verhinderung von Karies, Plaquebildung und von Gingivits. Derartige Zahnreinigungsmittel werden aber auch von Dentalhygienikern bei der beispielsweise halbjährlichen Zahnkontrolle beim Zahnarzt zum Reinigen der Zähne verwendet.

Zahnreinigungsmittel sind an sich bekannt und enthalten u.a. Silikate, Bimssteinkomponenten, Aluminiumtrioxid, Aluminiumsilikate, synthetisch hergestellte Fällungskieselsäure sowie Perlit, nebst weiteren Komponenten, wie Feuchthaltemittel, Benetzungsmittel, Verdicker, normalerweise in Zahnpasten verwendete Wirkstoffe wie Fluor sowie gegebenenfalls gingivitsverhütende Komponenten.

Wesentlich ist, dass das Zahnreinigungsmittel eine gute Reinigungswirkung aufweist, ohne jedoch den Zahnschmelz abzuradieren und ohne den Schmelz zu verkratzen. Auch soll bei der Anwendung des Zahnreinigungsmittels beim Zahnhalsansatz das Zahnfleisch nicht verletzt bzw. angegriffen werden. Aus der EP 0 268 763, der EP 0 528 756 sowie dem US-Patent 5 124 143 sind Zahnpflegemittel bekannt auf Basis synthetisch hergestellter Fällungskieselsäure sowie Perlit. Perlit ist ein Mineral vulkanischen Ursprungs. In der Mineralogie versteht man unter Perlit Liparit- und Quarz-Porphyrgläser, die eine kugelige Struktur aufweisen. Das Aufschäumen wird jedoch durch die Hitze und den Druck in der vulkanischen Umgebung bestimmt. Je nach Abbaugebiet ändert die chemische Zusammensetzung und der Reinheitsgrad. Teilweise sind in Perlit gar Schwermetalle nachweisbar. Die kugelförmigen Strukturen werden gemahlen, um die gewünschte Plättchenform zu erhalten, welche geeignet ist, Perlit in den vorgeschlagenen Zahnpflegemittelformulierungen zu verwenden. Eine weitere Eigenschaft liegt darin, dass die Partikelgrösse des Perlits variabel ist und teilweise Grössen bis zu 500 µm aufweist bei einem mittleren Teilchendurchmesser von ca. 60 bis 70 µm. Perlit muss also vor dem Einsatz in einem Zahnpflegemittel gesiebt werden. Die Aussiebung grosser Teilchen von über 200 µm verteuert das Produkt, und zudem hat es sich gezeigt, dass die üblicherweise im Handel verwendeten Produkte auf Basis von Perlit nach dem Ausspülen des Mundes einen sandigen Eindruck hinterlassen.

Es ist daher eine Aufgabe der vorliegenden Erfindung, ein Zahnreinigungsmittel der eingangs erwähnten Art zu schaffen, welches nicht auf der Basis von Perlit aufgebaut ist und welches trotzdem auf optimale Art und Weise den oben angeführten Anforderungen an ein derartiges Zahnreinigungsmittel gerecht wird.

Die gestellte Aufgabe wird mittels eines Zahnreinigungsmittels gemäss dem Wortlaut nach Anspruch 1 gelöst. Vorgeschlagen wird, dass das Zahnreinigungsmittel eine viskose Zusammensetzung aufweist, enthaltend gemahlenes Borosilikatglas. Mit Borosilikatglas oder auch C-Glas genannt werden Spezialgläser bezeichnet, welche nebst bis zu ca. 75 % Siliziumoxid Bortrioxid in der Grössenordnung von ca. 2 - 10 Gew% enthalten.

Gemäss einer bevorzugten Ausführungsvariante der Erfindung wird die Verwendung von C-Glas bzw. Alkalikalkglas mit erhöhtem Boroxidgehalt vorgeschlagen, welches an sich synthetisch hergestellt wird und insbesondere für Korrosionschutz-Rezepturen entwickelt worden ist. Das Herstellen und Aufschäumen dieses Borosilikatglases erfolgt unter bestimmten gesteuerten Bedingungen, wobei die chemische Zusammensetzung jeweils gleichbleibend ist. Die Zerkleinerung zur Herstellung der gewünschten Teilchengrösse, geeignet für die Verwendung im erfindungsgemäss definierten Zahnreinigungsmittel, erfolgt beispielsweise in Hammermühlen, und die Teilchengrösse ist standardisiert und liegt vorzugsweise unter 100 µm bei einem mittleren Teilchendurchmesser von ca. 10 - 20 µm, vorzugsweise ca. 15 µm. Diese auch als Microglas^{®} Glass Flakes bekannten Glaspartikel werden insbesondere auch in Beschichtungssystemen verwendet für die Herstellung eines hochqualitativen Korrosionsschutzes von Metall- und Betonuntergründen. In den beschriebenen Teilchengrössen ist das erfindungsgemäss beschriebene Borosilikatglass auch geeignet, ohne Vorbehandlung in die erwähnten Zahnreinigungsmittel eingearbeitet zu werden. Es handelt sich um Plättchen mit geometrischen Formen.

Demgegenüber ist die Partikelgrösse von Perlit viel variabler und beträgt bis zu 500 µm und mehr bei gleichzeitigem mittlerem Partikeldurchmesser von ca. 60 - 70 µm. Perlit müsste vor dem Einsatz ein einem Zahnreinigungsmittel zuerst gesiebt werden, und durch die Aussiebung der grösseren Teilchen von über 200 µm würde das Produkt sofort stark verteuert werden. Zusätzlich ist zu bemerken, dass grosse Partikel im Zahnschmelz oder Dentin eine tiefe Anrauhung hinterlassen, was selbstverständlich unerwünscht ist.

Die chemische Zusammensetzung von Borosilikatglas bzw. C-Glass weist je nach Qualität die nachfolgenden Wertebereiche auf:

| | |
|---|---|
| SiO₂ | 65 - 75 % |
| Al₂O₃ | 2 - 7 % |
| CaO | 4 - 9 % |
| MgO | 0 - 5 % |
| B₂O₃ | 2 - 10 % |
| Na₂O+K₂O | 9 - 13 % |
| ZnO | 1 - 6 % |

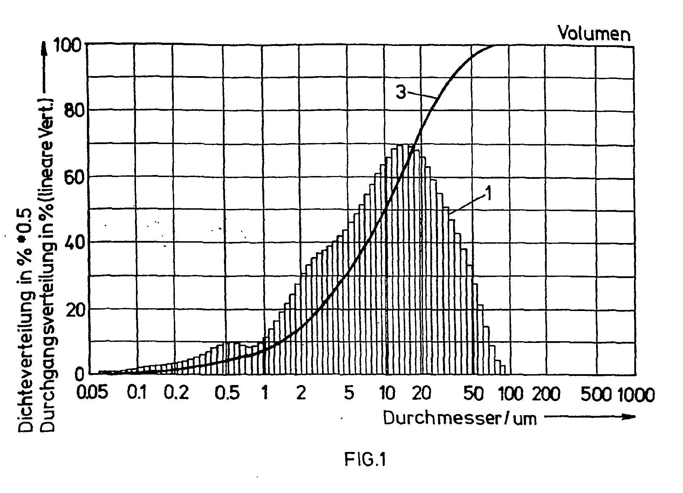

Als Borosilikatgläser bekannt sind u.a. Duran, Pyrex, Solidex oder das sogenannte FLAKEGLAS, welches bereits in gemahlener Form vorliegt und in der gewünschten Korngrössenverteilung bzw. mittleren Korngrösse kommerziell erhältlich ist. Eine typische Durchmesserverteilung des erfindungygemäss verwendeten Borosilikatglasses bzw. von Microglas^{®} Glass Flakes ist in der beigefügten Fig. 1 dargestellt. Dabei zeigen die Balken, bezeichnet mit dem Bezugszeichen 1, den mengenmässigen Anteil des jeweiligen Teilchendurchmesser, währenddem Linie 3 das Integral der Menge darstellt. Aus Fig. 1 deutlich erkennbar ist, dass keine Teilchen mit einem Durchmesser > 100 µm enthalten sind.

Nachfolgend werden anhand zweier Beispiele mögliche Rezepturen aufgelistet für die Herstellung eines Zahnreinigungsmittels unter Verwendung des erfindungsgemäss vorgeschlagenen Abrasivstoffes, des Borsilikatglasses bzw. der Microglas^{®} Glass Flakes.

### Beispiel 1, Rezeptur für eine Zahnpaste:

| | |
|---|---|
| Abrasivstoffe | ≤ 55 % |
| Verdickungsmittel | ≤ 2 % |
| Feuchthaltemittel | ≤ 60 % |
| Emulgatoren | ≤ 2 % |
| Fluoride | 0,05 - 0,15 % (maximal 1500 ppm) |
| Aromen/Süssstoffe | ≤ 2,5 % |
| weitere Zusatzstoffe | ≤ 1 % |

### Beispiel 2, Rezeptur für eine Prophylaxepaste:

| | |
|---|---|
| Abrasivstoffe | ≤ 65 % |
| Verdickungsmittel | ≤ 6 % |
| Feuchthaltemittel | ≤ 60 % |
| Emulgatoren | ≤ 5 % |
| Fluoride | ≤ 0,3 % |
| Aromen/Süssstoffe | ≤ 2,5 % |

Eine bevorzugte Rezeptur für die Herstellung sowohl einer Zahnpaste (ZP) wie auch einer Prophylaxepaste (PP) lautet wie folgt:

| | ZP in % | PP in % |
|---|---|---|
| Abrasivstoffe | ca. 10 - 20 | ca. 55 |
| Verdickungsmittel | ca. 0,5 | ca. 2 |
| Feuchthaltemittel | ca. 50 | ca. 30 |
| Emulgatoren | 1 ca. | ca. 1 |
| Fluoride | ca. 0,1 | ca. 0,1 |
| Aromen/Süssstoffe | ca. 1,2 | ca. 1,2 |

sowie im Falle von Zahnpasten noch ca. 0,1 % Zusatzstoffe. Beide Rezepturen werden mit Wasser bis auf 100 % ergänzt.

In den angeführten Rezepturen verhält sich das Borosilikatglas bzw. verhalten sich die Microglas^{®} Glass Flakes gegenüber allen Inhaltsstoffen inert. Eine zweijährige Lagerstabilitätsprüfung bei Raumtemperatur zeigt keine Veränderungen in und an der Paste. Der Fluorid- und pH-Wert bleiben stabil.

Der Konsument wünscht sich von einer Zahnpasta gut gereinigte, weisse Zähne, einen erfrischenden Geschmack und die Fluoridierung seiner Zähne. Den Preis, den der Kunde für die Erfüllung seines Wunsches bezahlt, ist der Verlust von Zahnhartsubstanz durch Abrasion und Anrauhung. Wohl weisen sowohl das aus dem Stand der Technik bekannte Perlit wie das erfindungsgemäss vorgeschlagene Borosilikatglass bzw. die Microglas^{®} Glass Flakes ein vergleichbares Verhalten auf bezüglich des Reinigungspotentials sowie des Abrasionsverhaltens, jedoch ergeben sich durch die Verwendung von Microglas^{®} Glass Flakes weniger Verkratzungen. Insbesondere aber die Herstellung einer Zahnpasta mit Microglas^{®} Glass Flakes ist einfacher, billiger und standardisierter als mit Perlit. Diese Vorteile spielen aber auch für den Hersteller eines Zahnreinigungsmittels, wie insbesondere einer Zahnpasta, eine grosse Rolle, auch im Hinblick auf die Produktehaftpflicht. Eine Zahnpasta bzw. genereller ein Zahnreinigungsmittel mit Microglas^{®} Glass Flakes ist für Personen mit freiliegendem Dentin im Bereich der Zahnhälse und mit Verfärbungen an den Zahnoberflächen infolge Rauchens bzw. häufigen Genusses von Kaffee, Tee usw. speziell gut geeignet. Für diese Konsumentengruppe ist die Auswahl an geeigneten Zahnpasten eher klein, womit das erfindungsgemäss vorgeschlagene Zahnreinigungsmittel eine gewichtige Lücke im Angebot schliesst. Ferner können Zahnpasten oder genereller Zahnreinigungsmittel entsprechend der erfindungsgemäss vorgeschlagenen Formulierung auch mit elektrischen Zahnbürsten problemlos angewendet werden.

## Patentansprüche

1. Zahnreinigungsmittel, **gekennzeichnet durch** eine viskose Zusammensetzung, enthaltend Plättchen mit geometrischen Formen mit einer Teilchengrösse < 200 µm und mittleren Durchmesser < 50 µm, bestehend aus Borosilikatglass der nachfolgenden Zusammensetzung:
| | |
|---|---|
| SiO₂ | 65 - 75 % |
| AI₂O₃ | 2 - 7 % |
| CaO | 4 - 9 % |
| MgO | 0 - 5 % |
| B₂O₃ | 2 - 10 % |
| Na₂O+K₂O | 9 - 13 % |
| ZnO | 1 - 6 % |

2. Zahnreinigungsmittel nach einem der Anspruch 1, **dadurch gekennzeichnet, dass** die Teilchengrösse des gemahlenen Glasses < 100 µm und der mittlere Durchmesser < 30 µm ist, vorzugsweise < 20 µm ist.

3. Zahnreinigungsmittel nach einem der Ansprüche 1 oder 2, **dadurch gekennzeichnet, dass** der Anteil Borosilikat ≤ 65 %, vorzugsweise 4 - 20 %, bezogen auf die gesamte Rezeptur, beträgt.

4. Zahnreinigungsmittel nach einem der Ansprüche 1 bis 3, **dadurch gekennzeichnet, dass** das Borosilikatglass oder auch genannt Alkalikalkglass bzw. C-Glass einen Bortrioxid-Gehalt aufweist in der Grössenordnung von 2 - 10 % und bevorzugt 2 - 7 %.

5. Zahnreinigungsmittel nach einem der Ansprüche 1 bis 4, **dadurch gekennzeichnet, dass** mindestens folgende Substanzen in der Rezeptur enthalten sind:
| | |
|---|---|
| Abrasivstoffe (Borosilikatglass) | ≤ 65 % |
| Verdickungsmittel | ≤ 7 % |
| Feuchthaltemittel | ≤ 61 % |
| Emulgatoren | ≤ 4 % |
| Fluoride % | 0,05 - 0,15 (max. 1500 ppm) |
| Aromen/Süssstoffe | 0,5 - 2 % |
| weitere Zusatzstoffe | 0 - 2 % |

6. Verwendung des Zahnreinigungsmittels nach einem der Ansprüche 1 bis 5 zur Herstellung einer Zahnpasta des täglichen Gebrauchs.

7. Verwendung des Zahnreinigungsmittels nach einem der Ansprüche 1 bis 5 zur Herstellung einer Zahnpasta für den wöchentlichen oder monatlichen Gebrauch.

8. Verwendung des Zahnreinigungsmittels nach einem der Ansprüche 1 bis 5 zur Herstellung einer Prophylaxepaste.

9. Verwendung des Zahnreinigungsmittels nach einem der Ansprüche 1 bis 5 zur Herstellung einer Zahnpasta, vorgesehen zum Reinigen von Zähnen bzw. zum Entfernen von Plaque und/oder zur Verhinderung von Karies.

## Claims

1. Teeth cleaning means, **characterized by** a viscous composition comprising small plates with geometric forms with a particle dimension of < 200 µm and an average diameter < 50 µm, consisting of borosilicate glass of the following composition:
| | |
|---|---|
| SiO₂ | 65 - 75 % |
| AI₂O₃ | 2 - 7 % |
| CaO | 4 - 9 % |
| MgO | 0 - 5 % |
| B₂O₃ | 2 - 10 % |
| Na₂O+K₂O | 9 - 13 % |
| ZnO | 1 - 6 % |

2. Teeth cleaning means according to claim 1, **characterized in that** the particle dimension of the ground glass is < 100 µm and the average diameter < 30 µm, preferably < 20 µm.

3. Teeth cleaning means according to one of the claims 1 or 2, **characterized in that** the content of borosilicate is ≤ 65 %, preferably 4 - 20 % based on the total recipe.

4. Teeth cleaning means according to one of the claims 1 to 3, **characterized in that** the borosilicate glass or also called alkali-lime glass or C-Glass respectively, does have a bortrioxid content in the amount of 2 - 10 % and preferably 2 - 7 %.

5. Teeth cleaning means according to one of the claims 1 to 4, **characterized in that** at least the following substances are contained within the recipe:
| | |
|---|---|
| Abrasive materials (borosilicate glass) | ≤ 65 % |
| Thickener | ≤ 7 % |
| Humidity maintaining means | ≤ 61 % |
| Emulgators | ≤ 4 % |
| Fluorides | 0,05 - 0,15 % (max. 1500 ppm) |
| Flavours/Sweeteners | 0,5 - 2 % |
| Further Additives | 0 - 2 % |

6. Use of the teeth cleaning means according to one of the claims 1 to 5 for the production of a toothpaste of the daily use.

7. Use of the teeth cleaning means according to one of the claims 1 to 5 for the production of a toothpaste for the weekly or monthly use.

8. Use of the teeth cleaning means according to one of the claims 1 to 5 for the production of a Prophylaxes paste.

9. Use of the teeth cleaning means according to one of the claims 1 to 5 for the production of a toothpaste designated for the cleaning of teeth or the removal of plaque and/or for the prevention of caries.

## Revendications

1. Agent d'hygiène dentaire, **caractérisé par** une composition visqueuse comprenant des plaquettes de forme géométrique avec une dimension de particule < 200 µm et un diamètre moyen < 50 µm, formées d'un verre de borosilicate avec la composition suivante:
| | |
|---|---|
| SiO₂ | 65 - 75 % |
| AI₂O₃ | 2 - 7 % |
| CaO | 4 - 9 % |
| MgO | 0 - 5 % |
| B₂O₃ | 2 - 10 % |
| Na₂O+K₂O | 9 - 13 % |
| Zno | 1 - 6 % |

2. Agent d'hygiène dentaire selon la revendication 1, **caractérisé en ce que** la dimension des particules du verre moulu est < 100 µm et le diamètre moyen est < 30 µm, de préférence < 20 µm.

3. Agent d'hygiène dentaire selon la revendication 1 ou 2, **caractérisé en ce que** le pourcentage du borosilicate est ≤ 65 %, de préférence 4 - 20 %, basé sur la recette totale.

4. Agent d'hygiène dentaire selon l'une des revendications 1 à 3, **caractérisé en ce que** le verre de borosilicate, appelé aussi verre sodico - calcique ou verre-C comprend un pourcentage en bortrioxyde de l'ordre de grandeur de 2 - 10 %, de préférence de 2 - 7 %.

5. Agent d'hygiène dentaire selon l'une des revendications 1 à 4, **caractérisé en ce que** la recette contient au moins les substances suivantes:
| | |
|---|---|
| Eléments abrasifs (verre de borosilicate) | ≤ 65 % |
| Agent épaississant | ≤ 7 % |
| Agent d'humidification | ≤ 61 % |
| Agent émulsionnant | ≤ 4 % |
| Fluorides | 0,05 - 0,15 % (max. 1500 ppm) |
| Agents aromatisants / agents sucrants | 0,5 - 2 % |
| Autres additifs | 0 - 2 % |

6. Utilisation de l'agent d'hygiène dentaire selon l'une des revendications 1 à 5 pour la fabrication d'une pâte dentifrice à utilisation journalière.

7. Utilisation de l'agent d'hygiène dentaire selon l'une des revendications 1 à 5 pour la fabrication d'une pâte dentifrice à utilisation hebdomadaire ou mensuelle.

8. Utilisation de l'agent d'hygiène dentaire selon l'une des revendications 1 à 5 pour la fabrication d'une pâte prophylactique.

9. Utilisation de l'agent d'hygiène dentaire selon l'une des revendications 1 à 5 pour la fabrication d'une pâte dentifrice, prévue pour nettoyer des dents ou pour enlever de la plaque et/ou pour empêcher la carie.
